# EUROPEAN PATENT APPLICATION

(11) **EP 1 230 934 A1**
(43) Date of publication of application: **14.08.2002**
(21) Application number: 00962973.4
(22) Date of filing: 29.09.2000
(51) Int. Cl.: A61K 47/34, C08J 3/02, C08G 69/40, B01J 13/00, A61K 48/00

(54) **POLYION COMPLEX MICELLES OF CORE/SHELL STRUCTURE**

(30) Priority: 19.11.1999 JP 33006599
(71) Applicant: NanoCarrier Co., Ltd., Kashiwa-shi, Chiba-ken 277-0882 (JP)
(72) Inventor: KATAOKA, Kazunori, Nakano-ku, Tokyo 165-0031 (JP); KAKIZAWA, Yoshinori, Bunkyo-ku, Tokyo 113-0021 (JP); HARADA, Atsushi, Yachiyo-shi, Chiba 276-0042 (JP)
(74) Representative: Albrecht, Thomas, Dr.
(86) International application number: JP0006761
(87) International publication number: WO0137879

(57) **Abstract**

Disclosed is a stabilizing method for polyion complex micelles having a core-shell structure which is formed from a block copolymer comprising a hydrophilic segment and a chargeable segment and a polyelectrolyte. Such stabilizing method can be achieved by endowing the chargeable segment in the block copolymer forming a core part with at least one thiol residue and allowing cross-links of disulfide bonds via the thiol residues endowed to them to be formed between the above chargeable segments. The representative of the copolymer which can be prepared by such stabilizing method can be represented by Formula (I), (II) or (III). Thus, capable of being provided is the stabilizing method for polyion complex micelles which can be utilized as a carrier for a polyelectrolyte.

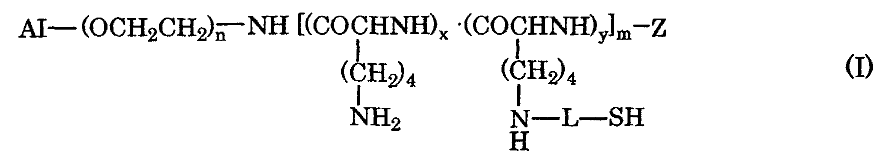

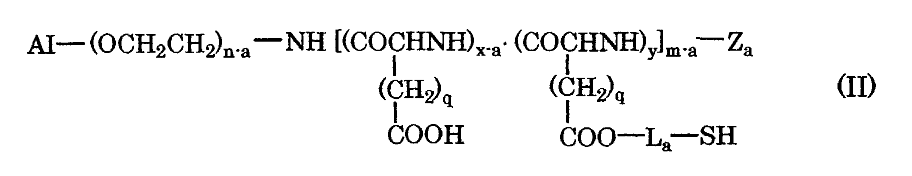

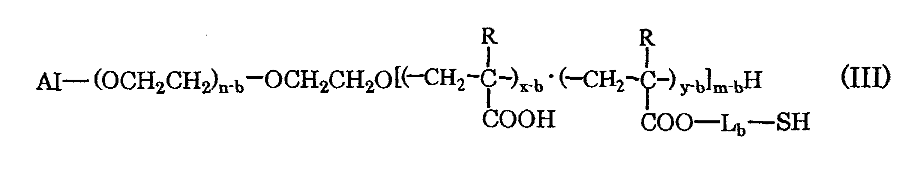

## Description

### Technical Field

The present invention relates to a stabilizing method for polyion complex micelles having a core-shell structure, the above stabilized micelles and a specific block copolymer which can be used for preparing the above micelles. In the above core-shell structure, the core part thereof comprises a chargeable segment of a block copolymer and a polyion complex of a polyelectrolyte, and the shell part comprises a hydrophilic segment of a block copolymer.

### Background Art

Poly(ethylene glycol)-block-poly(L-lysine) [hereinafter referred to as PEG-P(Lys)] which is a cationic block copolymer and a natural or synthetic anionic homopolymer are autonomously associated in water by virtue of an electrostatic interaction working between both to form spherical micelles. This micelle has a particle diameter of some ten nanometer and has a core-shell structure in which a polyion complex of an inner core is covered with a PEG crust, and it is called a polyion complex micelle (PIC micelle) (Harada and Kataoka, Macromolecules, 1995, 28, p. 5294 to 5299; Kataoka et al., Macromolecules, 1996, 29, p. 8556 to 8557; Harada et al., Macromolecules, 1998, 31, p. 288 to 294; and Kabanov et al., Macromolecules, 1996, 29, p. 6797 to 6802).

These PIC micelles can hold various anionic high polymers in an inner core covered with a crust of a PEG chain, and in addition thereto, they can be expected to avoid a foreign matter-recognizing system in organisms by virtue of a particle diameter of some ten nanometer and a core-shell structure. Accordingly, it is considered to be utilized, for example, as a carrier for anti-sense DNA and plasmid DNA used for gene therapy. In general, these PIC micelles are considerably stable under a physiological condition, but present in actual uses are cases where, after administration by intravenous injection, PIC micelles are dissociated by dilution, and are interacted with serum protein, and, thus, are not sufficiently stable under a physiological condition. This necessitates to modify the properties of PIC micelles so that they are stably present without being dissociated until they surely reach the target site. It is proposed to cause crosslinkage either in the inner nucleus (core) or in the crust (shell) by covalent bonding for the purpose of stabilizing PIC micelles (Guo et al., Macromolecules, 1996, 29, p. 2487 to 2493; and Thurmond et al., J. Am. Chem. Soc., 1996, 118, p. 7239 to 7240). However, in order to obtain the expected effect, cross-linking has to be cleaved in response to environment in the inside of the target cells to allow the PIC micelle to be dissociated to thereby release the DNA contained therein.

### Disclosure of the Invention

Accordingly, an object of the present invention is to provide such a stabilizing means for PIC micelles that PIC micelles are stable at least until they are delivered to an animal cell but readily dissociated in the cell.

The present inventors have found that when making use of cross-linking via a disulfide (-SS-) bond in place of cross-linking formed by a conventional covalent bond, PIC micelles can stably be maintained outside the cell but can readily be dissociated in the cell. It shall not theoretically be restricted, but such action and effect are considered to be attributable to the fact that in general, an animal cell stays in a strong reductive atmosphere in an inside thereof as compared with in blood flow (refer to, for example, Huang et al, Bioconjugate Chem., 1998, 9, p. 612 to 617), so that a disulfide bond is reduced to thiol in the cell and cleaved.

Accordingly, the present invention relates to a stabilizing method for polyion complex micelles having a core-shell structure which is formed from a block copolymer comprising a hydrophilic segment and a chargeable segment and a polyelectrolyte, wherein the chargeable segment in the block copolymer forming a core part is endowed with at least one thiol residue, and cross-links of disulfide bonds via the thiol residues endowed to them are formed between the above chargeable segments.

Further, the present invention relates to a polyion complex micelle composition comprising polyion complex micelles, which can be prepared by such stabilizing method as described above, having a core-shell structure formed from a block copolymer represented by Formula (I): 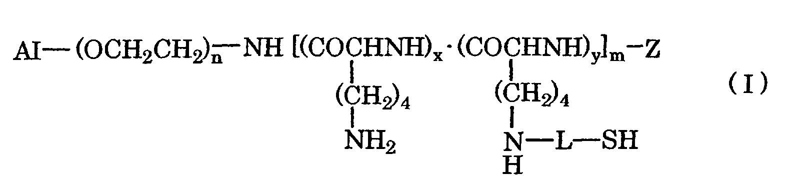 (wherein AI represents a hydrogen atom or an organic residue of an anionic polymerization initiator; Z represents a hydrogen atom or a residue of a formula ―L―SH; L represents a linkage group selected from the group consisting of C₁₋₂₀ alkylene, C₁₋₆ alkyl-phenyl, C₁₋₆ alkyl-phenylene-C₁₋₆ alkyl, phenylene and carbonyl-C₁₋₂₀ alkyl; n is an integer of 5 to 20,000 (preferably 50 to 20,000); m is an integer of 5 to 10,000; and a proportion of a unit of x to a unit of y is 20 : 1 to 1 : 2) and a polyelectrolyte, wherein a polyamine segment (the segment having repetitive units of m units in Formula (I)) in the above copolymer forming a core part and the polyelectrolyte form an ion pair in an aqueous medium; and two or more molecules in the above copolymer are cross-linked via at least one mercapto in the above segment.

On the other hand, the present invention using a copolymer of another embodiment relates to a stabilizing method for polyion complex micelles using a copolymer in which a polyamine segment in Formula (I) described above is substituted with polycarboxylic acid (for example, poly(aspartic acid), poly(glutamic acid), poly(methacrylic acid) or poly(acrylic acid)) and a polycarboxylic acid segment in which a part of them is turned into thiol, and the above a polyion complex micelle composition.

Further, the present invention relates to a block copolymer represented by Formula (I) described above.

### Brief Description of the Drawings

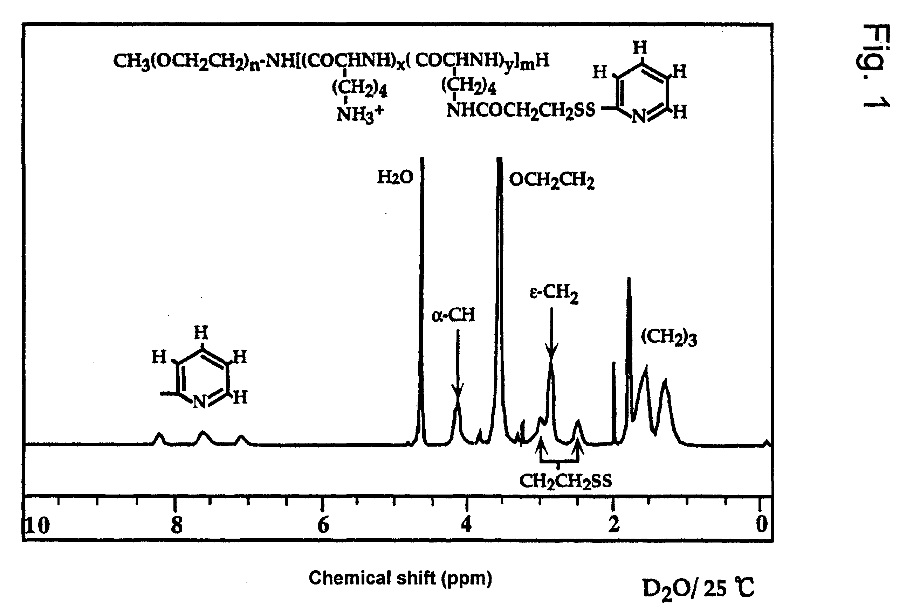

Fig. 1 is a ¹H-NMR spectrum of PEG-P(Lys) obtained in Production Example 1 into which a thiol residue is introduced.

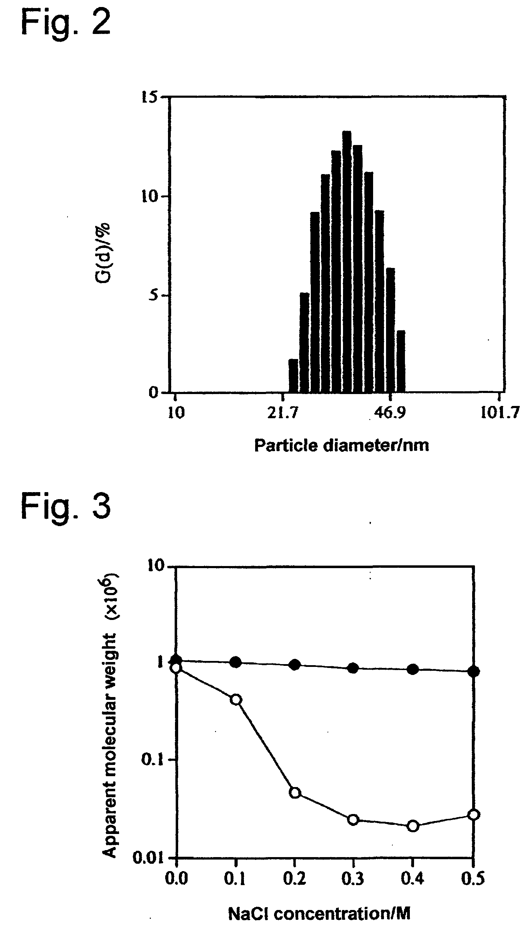

Fig. 2 is a graph showing a z-average particle diameter distribution of polyion complex micelles formed from PEG-P(Lys) obtained in Production Example 2 which is turned into thiol and P(Asp). It is based on the conditions of a measuring angle of 90°, a temperature of 25°C, a block copolymer concentration of 2 mg/ml and a solvent of 10 mM PBS (pH 7.4), 3 days after dialysis.
Fig. 3 is a graph showing a change in the apparent molecular weight of cross-linked micelles (black dots) and non-cross-linked micelles (void dots) to an NaCl concentration. It is based on the conditions of a block copolymer concentration of 1 mg/ml, a temperature of 25°C and a solvent of 10 mM PBS (pH 7.4).

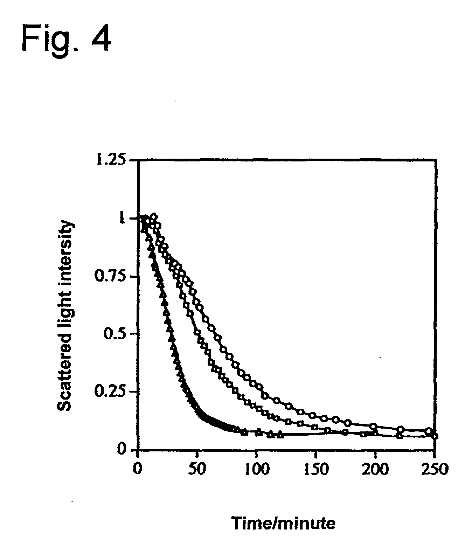

Fig. 4 is a graph showing a change in a scattered light intensity after adding dithiothreitol (DTT) to the PIC micelle solution according to the present invention. It is based on the conditions of DTT addition concentrations of 0.5 mM (circle), 1.0 mM (square) and 2.0 mM (triangle), a block copolymer concentration of 1.0 mg/ml, an NaCl concentration of 0.3M, a temperature of 25°C and a solvent of 10 mM PBS (pH 7.4).

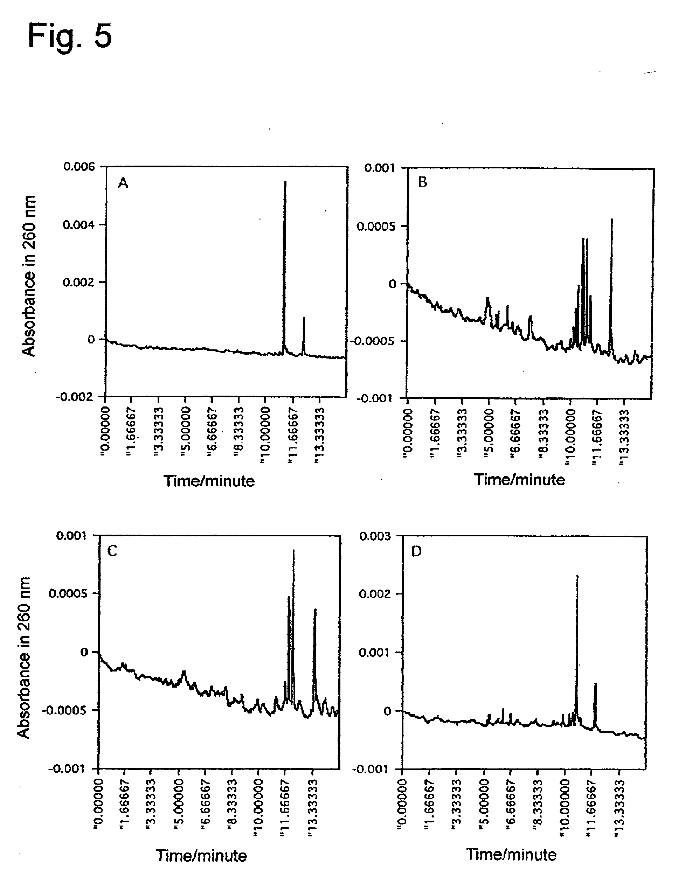

Fig. 5 is an electrogram showing the result of an enzyme resistance test of DNA in a DNA-contained cross-linked micelles obtained in Production Example 3 by capillary electrophoresis measurement. (A) is a solution of 20 mer DNA before enzyme treatment; (B) is a treated solution after one hour since free DNA enzyme treatment; (C) is a treated solution after one hour since non-cross-linked micelle enzyme treatment; and (D) is a treated solution after one hour since cross-linked micelles are treated by an engyme.

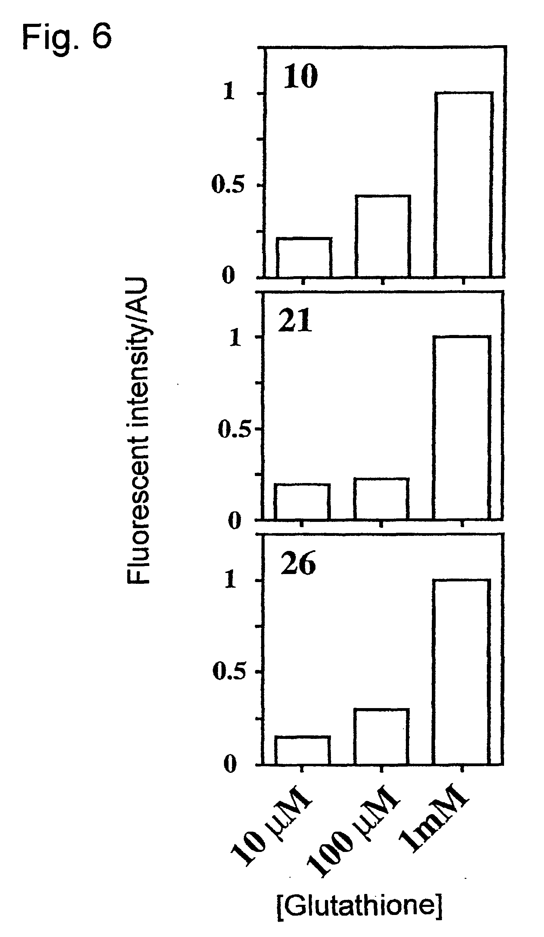

Fig. 6 is a graph showing the result of a release test of anti-sense DNA by dissociation of cross-linked micelles by means of GSH. Shown in (10) are the relative amounts of released DNA in a cross-linked micelle prepared using PEG-P(Lys) in which 10% of a lysine residue is turned into thiol; shown in (21) are the relative amounts of released DNA in a cross-linked micelle prepared using PEG-P(Lys) in which 21% of a lysine residue is turned into thiol; and shown in (26) are the relative amounts of discharged DNA in a cross-linked micelle prepared using PEG-P(Lys) in which 26% of a lysine residue is turned into thiol.

### Best Mode for Carrying Out the Invention

In the stabilizing method for polyion complex micelles having a core-shell structure according to the present invention, any block copolymers and polyelectrolytes used shall be included in the present invention without being restricted by the kinds thereof as long as the micelles are stabilized via a disulfide bond as described above. Capable of being used as such block copolymer are those obtained by subjecting a thiol residue to covalent bonding with a conventionally known copolymer comprising a hydrophilic segment and a chargeable segment by a suitable chemical method. The hydrophilic segment is preferably a segment comprising principally poly(ethylene glycol). In this case, the term "principally" means that poly(ethylene glycol) accounts for 80% or more in terms of a molecular weight ratio based on the segment. On the other hand, the chargeable segment means a segment containing in a repetitive unit, at least one group which can be charged either cationically or anionically in an aqueous medium (or an aqueous solution) having either pH, preferably pH 3.5 to 8.5. An amino group or an imino group and a quaternary amino group can be given as the cationically chargeable group, and these groups may be present on a high polymer side chain, or they may be present on a high polymer principal chain in the case of an imino group or a quaternary amino group. A carboxyl group, a sulfo group and a sulfuric acid group can be given as the anionically chargeable group, and these groups can be incorporated into a block copolymer by making use of conventionally known polymers having a repetitive unit comprising these groups as a high polymer side chain or making use of a production process for such polymer.

According to the present invention, these chargeable segments (including ω-terminals) have to be endowed with at least one thiol residue. In this respect, the thiol residue means a residue originating in a thiol compound, and these residues can be introduced by a conventional method using a mercapto group-containing alkylating agent or acylating agent which may be protected by, for example, the amino group or imino group described above or in the case of a carboxyl group, using mercapto group-containing amine or alcohol which may be protected by a carboxyl group.

Although not restrictive, the block copolymer in which a hydrophilic segment is poly(ethylene glycol) and in which a chargeable group in a chargeable segment is an amino group or an imino group includes PEG-(Lys) described in Kataoka et al., a poly(ethylene glycol)-polyspermine copolymer described in Kabanov et al., Bioconj. Chem., 1995, 6, p. 639 and a copolymer having a polyamine unit originating in oxazoline described in Japanese Patent Application No. 221026/1999 which is pending at the same time as the present application. A thiol residue can be introduced into the chargeable segments in these copolymers, for example, as described later in details, by using N-succinimdyl-3-(2-pyridyldithio)propionate of an active ester type and analogues thereof to acylate an amino group or an imino group and then reducing and cleaving a pyridyldithio group.

Accordingly, as a preferred embodiment of the present invention, stabilized PIC micelles using the block copolymer represented by Formula (I) described above are provided. The block copolymer itself represented by Formula (I) is a novel compound.

The organic residue originating in an anionic polymerization initiator specifying AI in the block copolymer represented by Formula (I) is, to be specific, a group represented by a formula: 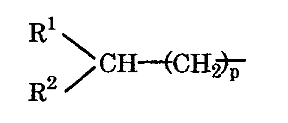 wherein:
(i) p is an integer of 0 to 10, and R¹ and R² each represent independently a hydrogen atom, C₁₋₁₀ alkoxy, aryloxy or aryl-C₁₋₃ alkyloxy, or R¹ and R² may be combined with each other to form ethylenedioxy (-O-CH(R')-CH-O-, in which R' is a hydrogen atom or C₁₋₆ alkyl) which may be substituted with C₁₋₆ alkyl, or an oxy (=O) group, or
(ii) p is 0 or 1, and R¹ and R² may be combined with each other to form an atomic group constituting a residue of monosaccharide or a derivative thereof.

A method for forming such AI―(OCH₂CH₂)̵ₙ segment is specifically described in, for example, Kataoka et al. described above, WO 96/32434, WO 96/33233 and WO 97/06202. These descriptions are incorporated into the present specification by citation. Further, a chargeable segment, for example, 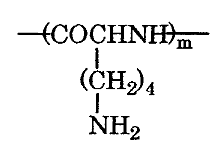 can be introduced into such hydrophilic segment as described above according to the methods described in details in Kataoka et al. and the literatures cited therein.

According to the present invention, the thiol residue is introduced through a covalent bond into the chargeable segment, for example, into the polyamine segment in the block copolymer according to the following reaction scheme: 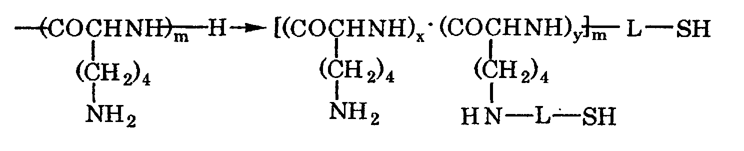 wherein L is a linkage group selected from the group consisting of C₁₋₂₀ alkylene, C₁₋₆ alkyl-phenyl, C₁₋₆ alkyl-phenylene-C₁₋₆ alkyl, phenylene and carbonyl-C₁₋₂₀ alkyl, preferably carbonyl-C₂₋₆ alkyl; an optimum proportion of a unit of x to a unit of y can be changed depending on an m value and therefore shall not be restricted, and it falls usually in a range of 20 : 1 to 1 : 2, further, the unit of x and the unit of y each may form a block but are preferably arranged at random.

The reaction scheme described above can be carried out according to a conventionally known reaction, for example, alkylation using corresponding monohalogenated thiol or acylation using a corresponding active ester.

The block copolymer having a thiol residue thus produced is a novel compound and is one embodiment of the present invention.

On the other hand, the block copolymer in which a hydrophilic segment is poly(ethylene glycol) and in which a chargeable group in a chargeable segment is a carboxyl group can be obtained by partially hydrolyzing a copolymer obtained by adding poly(β-benzylaspartic acid or γ-benzylglutamic acid) to poly(ethylene glycol) (refer to, for example, EP 0583 955B1) to remove a benzyl group and then introducing a thiol residue thereinto or by subjecting a benzyl group to transesterification with protected mercapto group-containing alcohol. Further, a thiol residue may be introduced into a poly(carboxylic acid) segment in a block copolymer obtained by further polymerizing poly(ethylene glycol) obtained by living cationic polymerization with methacrylic acid or acrylic acid (refer to, for example, WO 97/06202). A representative one of the copolymer thus obtained can be represented by the following Formulas (II) and (III): 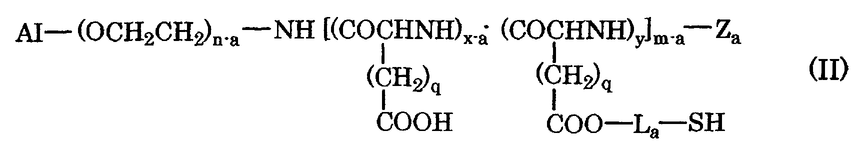 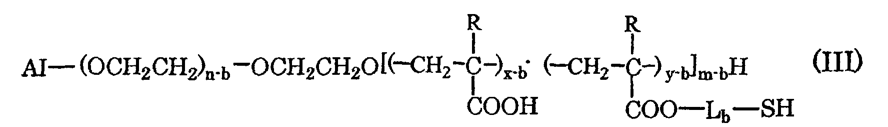 wherein respective AI's are the same as defined in Formula (I); Za represents a hydrogen atom or a residue of a formula ―La―SH or ―COOLa―SH; La and Lb each represent independently C₁₋₂₀ alkylene, C₁₋₆ alkyl-phenyl, C₁₋₆ alkyl-phenylene-C₁₋₆ alkyl or phenylene; R represents a hydrogen atom or methyl; n-a and n-b each represent independently an integer of 5 to 20,000; m-a and m-b each represent independently an integer of 5 to 10,000; q represents an integer of 1 or 2; and the proportions of a unit of x-a to a unit of y-a and a unit of x-b to a unit of y-b each are independently 20 : 1 to 1 : 2.

The PIC micelle according to the present invention can be prepared by treating the block copolymer which comprises a hydrophilic segment and a chargeable segment and in which the chargeable segment is endowed with at least one thiol residue and the poly electrolyte in an aqueous medium by the conventionally known method described in Harada and Kataoka or Kataoka et al. The specific examples of the treating method shall be explained in examples described later and therefore can be referred.

The polyelectrolyte contained in the PIC micelle of the present invention can be selected according to the chargeable segment and usually means a polymer having a group which is subjected to ionic dissociation in a water base medium (or water). However, it has to be noted that a prefix of "poly" is put to those falling under the category called a so-called oligomer in the present specification. Accordingly, polypeptide, oligopeptide, pseudopeptide, polynucleotide and oligonucleotide can be given as the polyelectrolyte, and they may be either linear or cyclic. Included in peptides are conventionally known various physiologically active peptides and physiologically active pseudopeptides, and DNA pieces, RNA pieces, anti-sense DNA and cyclic DNA are included in polynucleotides. These peptides and polynucleotides can cationically or anionically be charged in a certain case depending on pH, and therefore they can be used as well together with either of the block copolymer in which a chargeable group generates a cation and the block copolymer in which a chargeable group generates an anion by selecting conditions for preparing the PIC micelle. However, when using polynucleotides, the block copolymer generating a cation, particularly the block copolymer represented by Formula (I) is preferably used to prepare the PIC micelle.

The present invention is characterized by that after the PIC micelle is formed, cross-link is formed by a disulfide bond via a thiol residue endowed to a chargeable segment in the block copolymer. Such disulfide bond can be formed by leaving PIC micelles-containing an aqueous medium under an oxidizing condition. Usually, on the oxidizing condition, the medium is preferably left standing under an ambient atmosphere or a condition on which it is subjected to air oxidation. In general, the aqueous medium used in the present invention means water, a suitable buffer and in a certain case, an electrolyte and an aqueous solution containing a water-miscible organic solvent.

The stabilized PIC micelle according to the present invention can stably be present as well in an aqueous medium containing a considerable amount of an electrolyte but can be dissociated under a reducing condition particularly in an aqueous medium containing an electrolyte having a fixed concentration.

Thus, according to the present invention, capable of being provided is means suited for transporting or delivering particularly a polyelectrolyte to an organism.

The present invention shall further be explained below with reference to specific examples, and in order to make it easier to understand the present invention, the examples of a copolymer in which a poly(amine) segment is a poly(lysine) segment shall be given, but they shall not intend to restrict the present invention to them.

### Production Example 1: production of PEG-P(Lys) into which a thiol residue is introduced

### (1) Production of PEG-P(Lys)

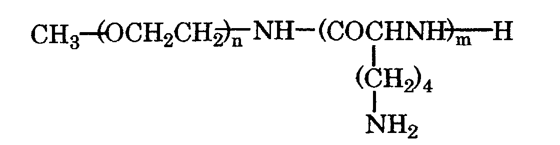

Used as an initiator was α-methoxy-ω-aminopoly(ethylene glycol) (PEG, MW = 5000) obtained according to the method described in Kataoka et al. described above to subject an N-carboxylic acid anhydride (NCA) of ε-benzyloxycarbonyl-L-lysine to ring-opening polymerization, whereby produced was poly(ethylene glycol)-block-poly(ε-benzyloxycarbonyl-L-lysine) (PEG-P(Lys(Z))). A benzyloxycarbonyl group (Z group) of PEG-P(Lys(Z)) thus obtained was deblocked with 30% HBr/AcOH to obtain PEG-P(Lys).

On the other hand, poly(aspartic acid) (P(Asp)) as a model compound for a polyelectrolyte was obtained by deblocking poly(β-benzyl-L-aspartate) obtained by polymerizing NCA of β-benzyl-L-aspartate by ring-opening polymerization.

The polymerization degrees of a polylysine segment of PEG-P(Lys) and P(Asp) determined by ¹H-NMR measurement were 22 and 15 respectively.

### (2) Introduction of thiol residue into PEG-P(Lys)

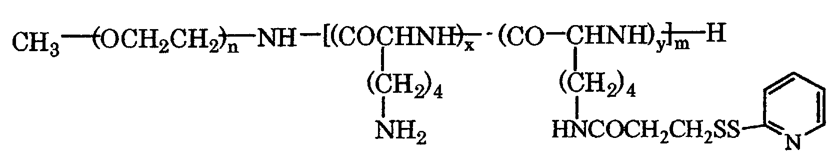

A pyridyldithiopropionyl group (PDP group) was introduced into PEG-P(Lys). This was introduced by using N-succinimdyl-3-(2-pyridyldithio)propionate (SPDP). Bromate of PEG-P(Lys) was dissolved in a 0.1N acetic acid buffer solution having a pH of 6.5, and the above buffer solution was dialyzed to exchange a paired ion with an acetic acid ion and then used. PEG-P(Lys) acetate (200 mg) and SPDP (56 mg, 0.5 mole equivalent based on a lysine residue) were dissolved in 5 ml of N-methylpyrrolidone (NMP, 5 wt% of lithium chloride was added, and the solution was deaerated). N,N-diisopropylethylamine of 0.5 ml was added to this solution in order to subject the amine to deprotonation to initiate reaction. The reaction solution was stirred at a room temperature for one hour, and the reaction was traced by reversed phase chromatography.

After finishing the reaction, the reaction solution was dropwise added to ether which was a poor solvent for PEG to reprecipitate it. An operation in which the crude product was dissolved in methanol and then reprecipitated in ether was repeated to remove impurities which were insoluble in water. The product was dissolved in a 0.1N acetic acid aqueous solution and dialyzed to distilled water for one hour to thereby remove excess salts. The final refined product was recovered by freeze-drying. The yield was 150 mg (the yield calculated from a mole number of the polymer was 69%).

The structure of the polymer thus obtained was confirmed by ¹H-NMR measurement (refer the spectrum to Fig. 1). A substitution degree of the PDP group was determined by ¹H-NMR measurement and UV measurement. In the ¹H-NMR measurement, D₂O was used as a solvent to determine the substitution degree from an intensity ratio in the peaks of proton (C₃H₄N: 7.6 ppm) of a pyridyl group in the PDP group and proton (OCH₂CH₂: 3.5 ppm) of a methylene group in PEG to find that it was 6.8. In the UV measurement, the substitution degree was calculated from an absorbance (λₘₐₓ = 343 nm, ε = 7.06 × 10³) of 2-thiopyridone which was liberated when the PDP group was reduced by dithiothreitol (DTT) to find that it was 6.7. The substitution degrees determined by two different methods were well congruous, and it was shown that the PDP group was introduced into 30% of an amino group in lysine.

### Production Example 2: production of PIC micelles in which an inner core was cross-linked by a disulfide (SS) bond

A PIC micelle in which an inner core was cross-linked by an SS bond was prepared by the following method. First, PEG-P(Lys) (PEG-P(Lys)-PDP) into which a PDP group was introduced by the method described above was dissolved in a 10 mM phosphoric acid buffer solution (pH 7.4), and then the solution was filtered through a filter of 0.1 µm to remove dusts. Next, DTT was added thereto so that the concentration thereof was as three times large as the PDP group, and the solution was stirred for 15 minutes to reduce the PDP group to a thiol residue. P(Asp) was added to this solution so that a ratio of a positive charge of PEG-P(Lys)-PDP to a negative charge of P(Asp) was 1 to thereby form a PIC micelle. In this respect, it was assumed based on the measurement results of PEG-P(Lys)-PDP and P(Asp) that all of the amino groups of PEG-P(Lys)-PDP and the carboxyl groups of P(Asp) were ionized.

Both polymers were mixed and left standing for 2 hours, and then the mixture was dialyzed to a 10 mM phosphoric acid buffer solution (pH 7.4) of 11 using a dialysis membrane having a cut-off molecular weight of 6000 to 8000 to remove DTT and 2-thiopyridone. The dialysis was continued for 3 days, and during the period thereof, thiol was oxidized to an SS bond by oxygen in the air, and it was cross-linked. After dialysis for 3 days, it was confirmed by an Ellman method that non-oxidized thiol was not present. The solution of the PIC micelles was transparent, and no precipitates were observed.

### Characteristic test: characteristic analysis of cross-linked micelles by light scattering method

The structure of the cross-linked PIC micelle was determined by a dynamic light scattering method (DLS). It was shown from results obtained by subjecting the micelle solution to DLS measurement to analyze the data by a histogram method that distribution in a particle diameter of the cross-linked PIC micelle was singly peaked and that the extent of the distribution was considerably small (dw/dn = 1.10; refer to Fig. 2). It was indicated that dependency of the scattering factor on the measuring angle was not found and that the micelles were spherical. Further, dependency of the scattering factor on the concentration was not found in the range of measurement (polymer concentration: 0.5 to 4.0 mg/ml), and secondary coagulation was not observed. A hydro-dynamic radius of the micelles was calculated from a value of the scattering factor determined by extrapolating to indefinite dilution using the equation of Stokes-Einstein to find that it was 16.1 nm. The polydispersibility determined by a cumulant method was 0.08, and it was a value equal to that of natural virus.

The micelles had an average particle diameter of 15.7 nm before oxidizing thiol to an SS bond before dialysis. The result that the PIC micelle has almost the same particle diameter before and after dialysis indicates that the formation of an SS bond does not exert a large effect on the structure of the micelle. No conspicuous change was observed on an average particle diameter of the PIC micelles at least for 2 weeks. This is considered due to the fact that the inner core of the PIC micelles is covered with a hydrophilic PEG layer and that they hold high stability as a colloidal particle because of steric repulsion.

The effect of the SS cross-linking on the stability of the PIC micelles was evaluated by measuring apparent molecular weight of the micelles against various NaCl concentrations by a static light scattering method (refer to Fig. 3). In the case of the PIC micelles into which thiol was not introduced and which was not cross-linked, the apparent molecular weight tended to be rapidly decreased as the NaCl concentration grew large, and the micelles were dissociated. On the other hand, a decrease in an apparent molecular weight of the cross-linked micelle was very small even in an NaCl concentration of 0.5M. Further, the cumulant particle diameter determined from DLS was 32.0 to 35.2 nm in the range of the NaCl concentrations used for evaluation, and it could be confirmed that the micelles were notably stabilized against the NaCl concentration.

In order to confirm that the PIC micelles were stabilized by the formation of cross-link in the SS bond, a reducing agent was added to trace a change in the scattered light intensity (refer to Fig. 4). If they are stabilized by virtue of the SS bond, addition of DTT which is a reducing agent selectively cleaving the SS bond under a high salt concentration is expected to dissociate the PIC micelles. The result obtained by actually tracing the scattered light intensity at a measuring angle of 90° after adding DTT showed a sudden reduction in the scattered light intensity. This reduction is considered to correspond to dissociation of the micelle, and it has been confirmed that the micelles are stabilized by the formation of the SS bond. Further, the dissociation speed is dependent on an addition concentration of DTT, and the micelles have completely been dissociated in about 50 minutes in a concentration of 2 mM. P(Asp) contained therein is considered to be released in the solution in line with the dissociation of these PIC micelles.

It is known that a reducing agent for an SS bond which is present in animal cells in the largest quantity is low molecular thiol called glutathione and that a concentration thereof is about 3 mM in a cell but is an order of 10 µM which is one three hundredth in a blood flow. Thus, the concentration is different to a large extent in and outside a cell, and since a dissociation speed of the SS bond depends on a concentration of a reducing agent, it is considered to be possible to make use of the PIC micelles stabilized by an SS bond as a carrier for a polyelectrolyte having a physiological activity including DNA, for example, polyanion into cells.

### Production Example 3: production of cross-linked PIC micelles containing therein anti-sense DNA

A PDP group was introduced into a lysine side chain of PEG-P(Lys) produced according to Production Example 1 according to Production Example 2 to obtain PEG-P(Lys) 12-39 (molecular weight of PEG: 12,000, a polymerization degree of lysine: 39 and an introduction rate of PDP: about 26% based on the lysine residue). This PDP group-introduced PEG-P(Lys) 12-39 was dissolved in a 25 mM Tris-HCl (pH 7.4) buffer solution, and then a reducing agent dithiothreitol (DTT) solution was added thereto to convert PDP into a thiol residue. This solution was blended with an anti-sense DNA (20 mer) solution (buffer solution: 25 mM Tris-HCl (pH 7.4)) so that a ratio of a charge of PEG-P(Lys) to that of DNA was 1 : 1 to form micelles. The solution was left standing for 3 hours or longer and then dialyzed to 25 mM Tris-HCl (pH 7.4) and 10 mM PBS (pH 7.4, 37°C) to remove impurities such as the reducing agent and oxidize and cross-link the inner core of the micelles by oxygen in the air. Characteristic test of DNA-containing cross-linked micelles obtained in Production Example 3:

### 1) Evaluation of decomposing enzyme resistance of anti-sense DNA by capillary electrophoresis measurement

### Experimental method:

Used as samples were the cross-linked micelle described above, non-cross-linked micelles prepared from PEG-P(Lys) 12-39 and anti-sense DNA (20 mer) and anti-sense DNA staying in a free state. A decomposing enzyme solution (enzyme: phosphodiesterase I: 0.02 unit) 110 µl was added to the respective solutions (DNA concentration: 196 µg/ml) 110 µl to react them at 37°C. After one hour, the solution was heated at 95°C for 5 minutes to deactivate the enzyme and terminate the reaction. Dextran sulfate, DTT and an internal standard 25 mer DNA solution 50 µl were added to the micelle solutions, which were used for measurement.

The following are conditions for capillary electrophoresis measurement.
Measuring apparatus: Bio Focus 3000 (Bio Rad Co., Ltd.)
Capillary: coated capillary (75 µm ID × 24 cm)
Loaded voltage: 12 kV
Migration liquid: high viscous liquid for
oligonucleotide (manufactured by Bio Rad Co., Ltd.)
Sample injection: 10 kV, 3 seconds
Detection: 26 nm

### Experimental results:

Shown in Fig. 5 are the electroferrograms of 20 mer anti-sense DNA and of internal standard 20 mer DNA before enzyme treatment, free DNA subjected to enzyme treatment, non-cross-linked micelles subjected to enzyme treatment and an anti-sense DNA-containing cross-linked micelle subjected to enzyme treatment. A peak of 20 mer almost disappears one hour later in free anti-sense DNA, but DNA contained in the micelles is inhibited from being decomposed. It is confirmed by comparison of the electroferrograms of the non-cross-linked micelles and the cross-linked micelles observed after reaction for one hour that decomposition products are scarcely found in the case of the cross-linked micelles and that the resistance against a decomposing enzyme for DNA is further elevated.

### 2) Dissociation of cross-linked micelles by an intercellular reducing agent glutathione (GSH)

### Experimental method:

Used for cross-linked micelles were micelles prepared by using PEG-P(Lys) in which thiol groups of 10, 21 and 26% were introduced into a lysine residue. Cross-linked micelles were prepared by the method described above. GSH solutions (GSH concentrations: OM. 20mM, 200mM and 2mM) to which polyvinyl sulfate equivalent to DNA were added to the respective micelle solutions (DNA concentration: 294 µg/ml) 20 µl, and the solutions were left standing at 25°C for 24 hours. The released anti-sense DNA was separated by 12.5% polyacrylamide electrophoresis and determined. DNA was dyed by etidium bromide. Electrophoresis was carried out at a loaded voltage of 300 V and an electrophoresis time of 30 minutes. A band of the released DNA was determined by means of an image analytical apparatus.

### Experimental results:

Shown in Fig. 6 are graphs obtained by determining a band of free DNA obtained by electrophoresis by means of an image processing apparatus and showing it in the form of a bar graph, wherein an amount of DNA released in the state of a GSH concentration of 1 mM was set to 1. In the respective cross-linked micelles prepared by using PEG-P(Lys) into which thiol groups of 10, 21 and 26% were introduced, it was shown that in the cases of the GSH concentrations of 100 µM and 10 µM as compared with a GSH concentration of 1 mM, the DNA-releasing amount was small and the micelle was dissociated dependently on the concentration of GSH and that DNA contained therein was released. It is known that the concentration of GSH is several mM in a cell and 10 µM in a blood flow, and therefore the results described above indicate that the inner core cross-linked micelles formed by an SS bond is selectively dissociated in cells.

### Industrial Applicability

The inner core cross-linked micelles formed by an SS bond according to the present invention can selectively be dissociated under reducing atmosphere such as an inside of cells. Accordingly, it is advantageous, for example, for specifically delivering a chemical under such atmosphere. Thus, the present invention can be used in the formulation industry and the like.

## Claims

1. A stabilizing method for polyion complex micelles having a core-shell structure which is formed from a block copolymer comprising a hydrophilic segment and a chargeable segment and a polyelectrolyte, wherein the chargeable segment in the block copolymer forming a core part is endowed with at least one thiol residue, and cross-links of disulfide bonds via the thiol residues endowed to them are formed between the above chargeable segments.

2. The stabilizing method as described in claim 1, wherein the hydrophilic segment in the block copolymer comprises poly(ethylene glycol); the chargeable segment comprises polyamine or polycarboxylic acid; and the polyelectrolyte is selected from the group consisting of polypeptide, polypseudopeptide and polynucleotide.

3. The stabilizing method as described in claim 1, wherein the hydrophilic segment comprises poly(ethylene glycol), and the chargeable segment comprises polyamine.

4. The stabilizing method as described in claim 1, wherein the hydrophilic segment comprises poly(ethylene glycol), and the chargeable segment comprises polycarboxylic acid.

5. The stabilizing method as described in claim 1, wherein the block copolymer is represented by Formula (I): 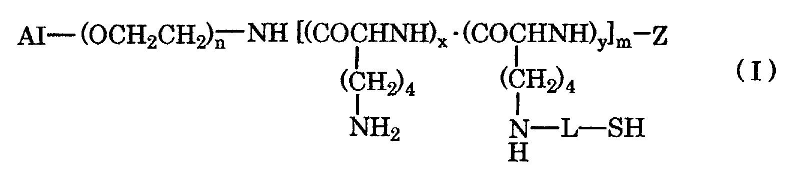 wherein AI represents a hydrogen atom or an organic residue of an anionic polymerization initiator; Z represents a hydrogen atom or a residue of a formula ―L―SH; L represents a linkage group selected from the group consisting of C₁₋₂₀ alkylene, C₁₋₆ alkyl-phenyl, C₁₋₆ alkyl-phenylene-C₁₋₆ alkyl, phenylene and carbonyl-C₁₋₂₀ alkyl; n is an integer of 5 to 20,000; m is an integer of 5 to 10,000; and a proportion of a unit of x to a unit of y is 20 : 1 to 1 : 2.

6. The stabilizing method as described in claim 1, wherein the block copolymer is represented by Formula (II) or (III): 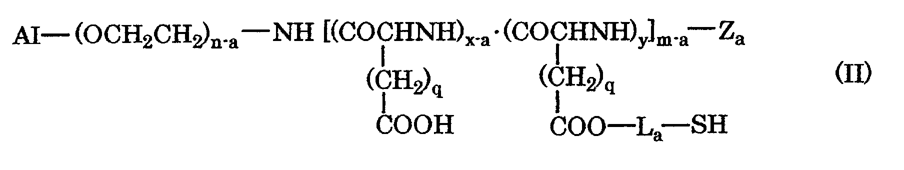 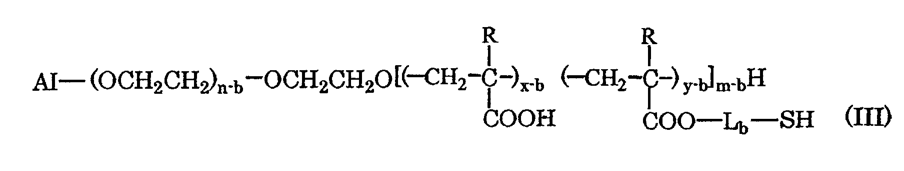 wherein respective AI's are the same as defined in Formula (I); Za represents a hydrogen atom or a residue of a formula ―La―SH or ―COLa―SH; La and Lb each represent independently C₁₋₂₀ alkylene, C₁₋₆ alkyl-phenyl, C₁₋₆ alkyl-phenylene-C₁₋₆ alkyl or phenylene; R represents a hydrogen atom or methyl; n-a and n-b each represent independently an integer of 5 to 20,000; m-a and m-b each represent independently an integer of 5 to 10,000; q represents an integer of 1 or 2; and the proportions of a unit of x-a to a unit of y-a and a unit of x-b to a unit of y-b each are independently 20 : 1 to 1 : 2.

7. Polyion complex micelles composition comprising polyion complex micelles having a core-shell structure formed from a block copolymer represented by Formula (I): 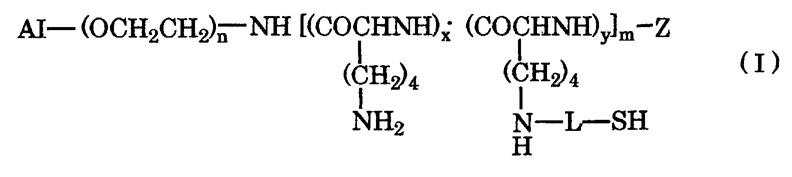 (wherein AI represents a hydrogen atom or an organic residue of an anionic polymerization initiator; Z represents a hydrogen atom or a residue of a formula ―L―SH; L represents a linkage group selected from the group consisting of C₁₋₂₀ alkylene, C₁₋₆ alkyl-phenyl, C₁₋₆ alkyl-phenylene-C₁₋₆ alkyl, phenylene and carbonyl-C₁₋₂₀ alkyl; n is an integer of 5 to 20,000; m is an integer of 5 to 10,000; and a proportion of a unit of x to a unit of y is 20 : 1 to 1 : 2) and a polyelectrolyte, wherein a polyamine segment (the segment having repetitive units of m units in Formula (I)) in the above block copolymer forming a core part and the polyelectrolyte form an ion pair in an aqueous medium; and the above copolymer is cross-linked via at least one thiol residue in the above segment.

8. The composition as described in claim 7, wherein the polyelectrolyte is an oligomer or polynucleotide.

9. The composition as described in claim 7, wherein the polyelectrolyte is an anionically chargeable oligomer or polypeptide.

10. A block copolymer represented by Formula (I): 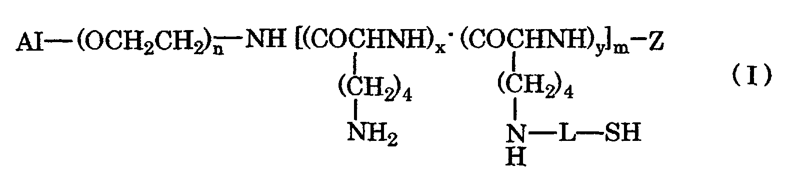 wherein AI represents a hydrogen atom or an organic residue of an anionic polymerization initiator; Z represents a hydrogen atom or a residue of a formula ―L―SH; L represents a linkage group selected from the group consisting of C₁₋₂₀ alkylene, C₁₋₆ alkyl-phenyl, C₁₋₆ alkyl-phenylene-C₁₋₆ alkyl, phenylene and carbonyl-C₁₋₂₀ alkyl; n is an integer of 5 to 20,000; m is an integer of 5 to 10,000; and a proportion of a unit of x to a unit of y is 20 : 1 to 1 : 2.

11. The block copolymer as described in claim 8, wherein AI in Formula (I) is a hydrogen atom or a group represented by a formula: 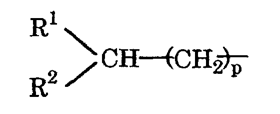 wherein:
(i) p is an integer of 0 to 10, and R¹ and R² each represent independently a hydrogen atom, C₁₋₁₀ alkoxy, aryloxy or aryl-C₁₋₃ alkyloxy, or R¹ and R² may be combined with each other to form ethylenedioxy (-O-CH(R')-CH-O-, in which R' is a hydrogen atom or C₁₋₆ alkyl) which may be substituted with C₁₋₆ alkyl, or an oxy (=O) group, or
(ii) p is 0 or 1, and R¹ and R² may be combined with each other to form an atomic group constituting a residue of monosaccharide or a derivative thereof.

12. The block copolymer as described in claim 10 or 11, wherein L in Formula (I) is carbonyl-C₂₋₆ alkyl.
